Europäisches Patentamt

European Patent Office

Office européen des brevets

(19)

(11) **EP 1 332 006 B1**

(12) **EUROPEAN PATENT SPECIFICATION**

(45) Date of publication and mention
of the grant of the patent:
**15.09.2004 Bulletin 2004/38**

(51) Int Cl.7: **B05B 17/06**, A61M 15/00,
B05B 12/08

(21) Application number: **01974597.5**

(22) Date of filing: **12.10.2001**

(86) International application number:
**PCT/IB2001/001924**

(87) International publication number:
**WO 2002/036269 (10.05.2002 Gazette 2002/19)**

(54) **DEVICE AND METHOD FOR DETECTING AND CONTROLLING LIQUID SUPPLY TO AN APPARATUS DISCHARGING LIQUID**

VORRICHTUNG UND VERFAHREN ZUM ERFASSEN UND REGELN DER FLÜSSIGKEITSVERSORGUNG ZU EINEM FLÜSSIGKEITSAUSSTOSSAPPARAT

PROCEDE ET DISPOSITIF DE DETECTION ET DE COMMANDE DE L'ALIMENTATION EN LIQUIDE D'UN APPAREIL D'EVACUATION DE LIQUIDE

(84) Designated Contracting States:
**AT BE CH CY DE DK ES FI FR GB GR IE IT LI LU MC NL PT SE TR**

(30) Priority: **30.10.2000 US 699049**

(43) Date of publication of application:
**06.08.2003 Bulletin 2003/32**

(73) Proprietor: **Instrumentarium Corporation**
**00670 Helsinki (FI)**

(72) Inventor: **HAVERI, Heikki**
**FIN-00360 Helsinki (FI)**

(74) Representative: **Charlton, Peter John**
**Elkington and Fife LLP,**
**Prospect House,**
**8 Pembroke Road**
**Sevenoaks, Kent TN13 1XR (GB)**

(56) References cited:
WO-A-01/19437          US-A- 3 164 990
US-A- 4 283 945        US-A- 5 487 378
US-A- 5 586 550        US-A- 5 938 117
US-A- 6 048 328

**Description**

BACKGROUND OF THE INVENTION

The present invention relates to an improved apparatus, such as a nebulizer apparatus, discharging liquid. Nebulizers, or atomizers, are devices that generate a fine spray or aerosol, usually of liquid. A particularly useful application for nebulizers is to provide a fine spray containing a dissolved or a suspended particulate or colloidal pharmaceutical agent for administration to a subject by inhalation. Such inhalation treatment is highly effective for conditions affecting the subject's respiratory organs. Further, since the lungs are close to the heart and the blood circulatory system of the body, drug administration by inhalation provides an effective and rapid delivery system to all organs of the body. Other applications include dispensing insecticides, paint, deodorants, water for humidification, etc. Other apparatuses which may incorporate the present invention include printers in which ink is discharged onto paper.

When dispensing a pharmaceutical agent, in many cases, a nebulizer is placed directly in the mouth or nose of the subject so that the spray can be entrained in the respiratory gases inhaled during normal, spontaneous breathing of the subject. In other cases, the subject breathes with the aid of a respiratory ventilator. A typical ventilator has a breathing circuit comprising an inhalation limb and an exhalation limb connected to two arms of an Y-connector. The third arm of the Y-connector is connected, via a patient limb, to a mouthpiece, mask or endotracheal tube for the subject. The ventilator provides a complete or partial supply of respiratory gases to the subject through the inhalation limb during inhalation. The contraction of the subject's lungs discharges gas through the exhalation limb during exhalation. When a nebulizer is employed in conjunction with a ventilator, it is typically placed in the patient limb but can also be placed in the inhalation limb.

Nebulizers currently in use for ventilator applications generate the spray either pneumatically or by means of ultrasonic vibrations. Pneumatic nebulizers are typically used with a liquid, such as an aqueous drug solution. High pressure driving gas is conducted through a nozzle to draw the drug from a drug supply for the nebulizer. The drug is discharged against a baffle or other means in a gas space of the nebulizer, breaking the liquid into a fine spray. The gas space is in fluid communication with the inhaled gas pathway of the breathing circuit so that the gas flow expelled from the nozzle along with the nebulized drug is conducted to the breathing circuit and ultimately to the subj ect.

Disadvantages in the use of pneumatic nebulizers include the following. If the nebulizer adds a significant quantity of gas, for example, up to five liters/minute, into the breathing circuit, the breathing gas composition may be affected. Due to passage of the driving gas through the nozzle, impingement of the drug on the baffle, etc., pneumatic nebulizers are noisy. Also, controlling the commencing and stopping of a drug agent spray is difficult and not very accurate, resulting in wastage of the drug.

The foregoing shortcomings of pneumatic nebulizer have led to the use of ultrasonic nebulizers employing a vibrating element, such as a piezoelectric crystal. Breathing gas composition and the on-off operation are easier to control with such nebulizers than in a pneumatic nebulizer. However, ultrasonic devices may require a large, bulky electrical power supply to power the ultrasonic vibrator and may not be able to nebulize colloidal or particulate suspensions.

In one type of ultrasonic nebulizer, the fine spray is produced dropping the liquid on, or otherwise applying it to, the vibrating element. See U.S. Patent 5,443,059. U.S. Patent 3,812,854 describes another type of nebulizer, for use in inhalation therapy, in which the spray is generated on the front surface of a vibrating, porous body. The pores of the body form a network of passages that enable the liquid to flow through the body. The liquid to be nebulized is supplied under pressure to the pores on the rear surface of the body, and forced through the pores to the front surface of the porous body where it is discharged as a spray. U.S. Patent 5,487,378 describes a nebulizer in which the aerosol is formed using a mesh plate instead of a porous solid body. The mesh plate has a plurality of orifices for the liquid. The liquid or the nozzle assembly is vibrated ultrasonically by a piezoelectric element to nebulize a dose of liquid as it passes through the mesh plate. The supply of each dose through the nebulizer is sensed by a dose gauge.

A specific difficulty with nebulizers in which the liquid or the orifice assembly is vibrated ultrasonically, as by a piezoelectric element, to nebulize the liquid is control of the supply of liquid to the nebulizer so that the right amount necessary for proper operation is present in the nebulizer.

U.S. Patents 5, 518,179 and 5,299,739 describe nebulizers in which capillary feed is used to supply liquid to the vibrating element. A further alternative for liquid supply is achieved by condensing a liquid vapor on one face of the membrane, the liquid thus condensed being dispensed in droplet form. See U.S. Patent 5,518,179.

U.S. Patent 5,938,117 describes an apparatus for dispensing liquids as an atomized spray and having a fluid supply system that transports fluid to an apertured oscillating surface. The fluid supply system is connected to an electronic flow control valve. The valve is connected to an electronic circuit. In the event of excessive delivery of liquid, the oscillation amplitude decreases and the current draw by the piezoelectric element decreases. A current sensor senses the reduced current draw and transmits an overflow signal to the flow control valve to reduce the delivery rate of liquid to the surface until the amount of fluid returns to a normal level.

Document WO 119 437, which has not been published before filing date discloses an apparatus analogous to that of the present application except for the fact that it does not include means for measuring capacitance existing between two spaced apart member.

## BRIEF SUMMARY OF THE INVENTION

An object of the present invention is to provide an improved device and method for accurately controlling the supply of liquid in an apparatus discharging liquid. One such apparatus can comprise a nebulizer in which the accurate liquid supply enables the liquid to efficiently transformed into an aerosol. The invention may be employed with other types of apparatuses, such as those discharging ink for printing purposes.

The invention is particularly suited for use with a nebulizer employing an ultrasonically vibrating element but can also be extended to other type of nebulizers in which proper functioning and efficiency are dependent on control of the liquid into the nebulizer.

The above objects are obtained by an improved device and method for measuring the amount of liquid to be discharged that is present in the apparatus discharging the liquid. To this end, in a typical embodiment of the invention, a nebulizer includes a first member having holes through which the liquid passes to be nebulized. A second member is spaced from the first member so that a volume is defined in the nebulizer by the area of mutual overlap of the first and second members and the amount of spacing between them in the area of overlap. The first and second members have electrically conductive properties, as by being formed of conductive material or having an electrically conductive coating. The first and second members are electrically isolated from each other. The liquid to be nebulized is provided into the volume between the first and second members and circuitry is coupled to the first and second members to measure the capacitance between the members. The capacitance between the members indicates the amount of liquid in the volume defined in the nebulizer. A vibrator, such as a piezoelectric element, vibrates the liquid, as by bowing one of the first and second members, to carry out the nebulization of the liquid. The capacitance measuring circuit may be coupled to a liquid supply to, cause the latter to, preferably intermittently, provide additional liquid to the volume as the nebulization of the liquid proceeds.

Various other features, objects, and advantages of the invention will be made apparent from the following detailed description and the drawings.

## BRIEF DESCRIPTION OF THE SEVERAL VIEWS OF THE DRAWING

The foregoing objects and advantages, as well as the invention itself, will be more fully understood from the attached drawing and following detailed description.

In the drawings:

Fig. 1 is a general cross sectioned view of a nebulizer apparatus in which the present invention may be used, the operational environment for the nebulizer apparatus being shown in a generalized schematic form;
Fig. 2a is an exploded, partial, cross sectional view showing the apparatus of Fig. 1;
Figs. 2b and 2c are schematic views showing the operation of the apparatus of Fig. 1;
Fig. 3 is a detailed view of certain elements used for detecting liquid supply by capacitive measurement;
Fig. 4 is a three dimensional portrayal representing the behavior of capacitance in the capacitive measurement employed in the present invention;
Fig. 5 is a schematic diagram of electrical circuitry for measuring capacitance;
Fig. 6 is a schematic view representing an alternative construction for the present invention; and
Fig. 7 is a schematic view representing another alternative construction of the present invention.

## DETAILED DESCRIPTION OF THE INVENTION

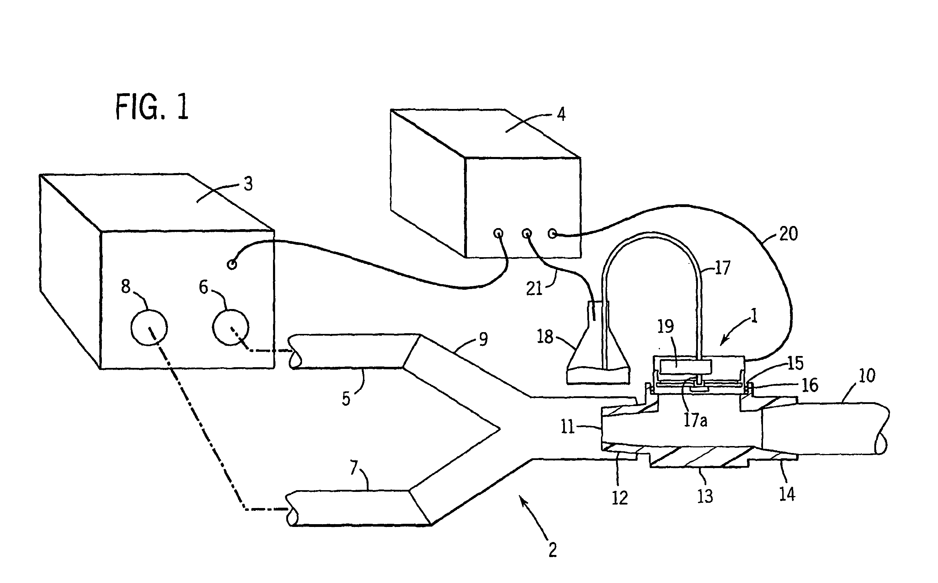

Apparatus 1, such as a nebulizer apparatus, incorporating the present invention is typically used in conjunction with breathing circuit 2, ventilator 3 and control unit 4, as shown in Fig. 1. The nebulizer 1 atomizes liquid solutions or suspensions for delivery to a subject, as for example as a drug treatment for a patient. Breathing circuit 2 includes inhalation limb 5, which is coupled to ventilator 3 at inhalation limb connector 6. Exhalation limb 7 is connected to ventilator 3 at exhalation limb connector 8. Inhalation limb 5 and exhalation limb 7 are connected to two arms of Y-connector 9. A third arm of Y-connector 9 is connected to one end of patient limb 10. The other end of patient limb 10 is directed to a mouthpiece, facemask, or endotracheal tube for the subject

Ventilator 3 provides all or a portion of the respiratory gases for the subject by providing inhalation gases in inhalation limb 5. The inhalation gases pass through Y-connector 9 and into patient limb 10 for supply to the subject. On exhalation, the respiratory gases pass through patient limb 10, Y-connector 9, and exhalation limb 7 back to ventilator 3.

As shown in Fig. 1, the nebulizer apparatus 1 is preferably positioned in patient breathing circuit 2 as near the patient as possible to minimize the aerosol transport path, and to minimize the deposition of the aerosol on the breathing circuit walls. To this end, nebulizer apparatus 1 may be inserted in the breathing circuit between Y-connector 9 and patient limb 10. The Y-connector 9 has a socket 11 for receiving tubular projection 12 of adapter 13 for nebulizer apparatus 1. The tubular socket 14 of the adapter 13 receives the patient limb 10. The nebulizer apparatus is placed in opening 15 in adapter 13 and held in place with O-ring seal 16. When nebulizer apparatus 1 is not needed, or when the nebulizer apparatus is removed for cleaning or maintenance, a cap (not shown) may be fitted into or over the opening 15 to allow breathing circuit 2 to function in a normal manner. Alternatively, the entire adapter 13 containing nebulizer apparatus 1 may be removed from the breathing circuit and patient limb 10 reconnected to Y-connector 9. Control unit 4 for the nebulizer apparatus may be located at a distance from nebulizer apparatus 1 and may be incorporated in ventilator 3, if desired.

Nebulizer apparatus 1 is connected to a source of material to be nebulized. In the embodiment shown in Fig. 1, conduit 17 and transport line 17a supply material from reservoir 18 to apparatus 1. Reservoir 18 can be placed at a desired location and can be proximate to, or remote from, nebulizer apparatus 1. A control valve 19 is provided between the supply conduit 17 and the transport line 17a. Electrical control signals are supplied to control valve 19 via cable 20 from control unit 4. The material to be nebulized is usually a liquid and can comprise a solution, or a particulate or colloidal suspension, of a pharmaceutical agent For purposes of explanation, the material undergoing nebulization is hereinafter generally described as a liquid. Reservoir 18 is pressurized by pump in control unit 4. In the embodiment shown in Fig. 1, a pump inside control unit 4 supplies a pressurizing gas to reservoir 18 through pressure line 21.

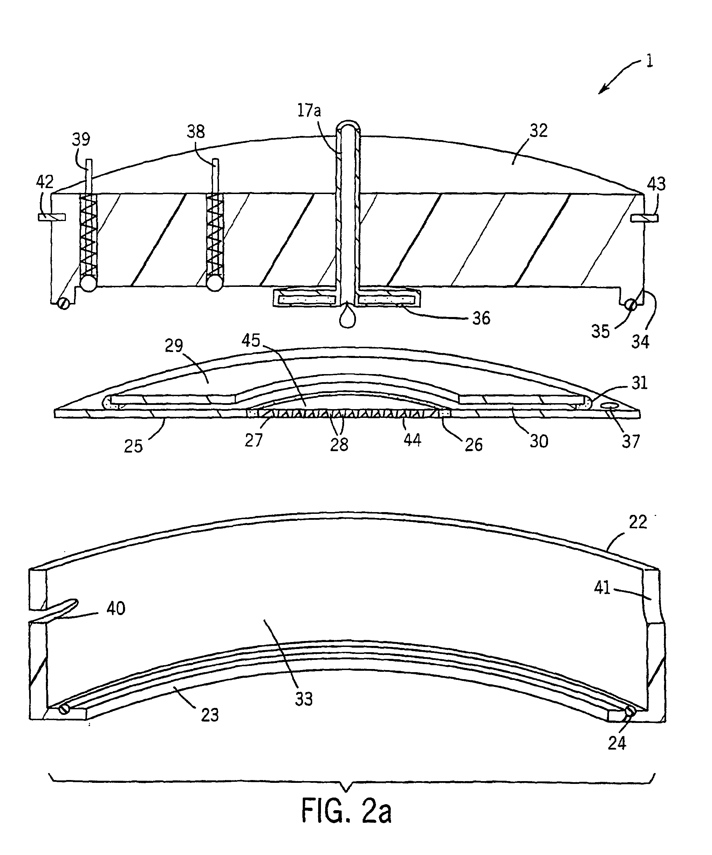

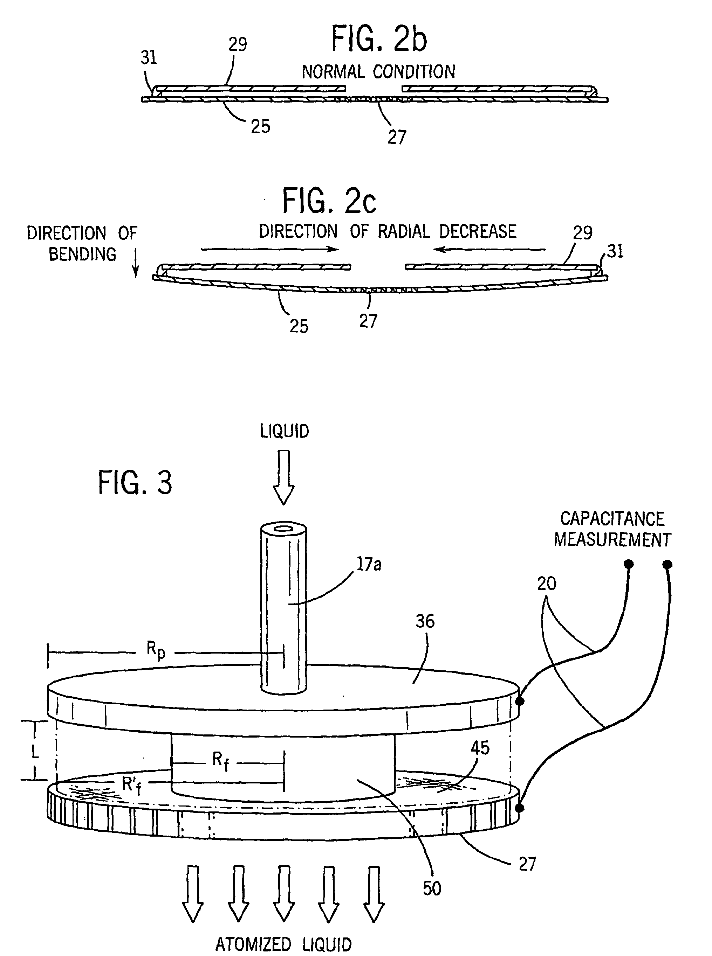

Nebulizer apparatus 1, is shown in detail in Figs. 2a, 2b, 2c. Nebulizer apparatus 1 shown in the cross sectional, exploded view of Fig. 2a has annular housing 22, which mounts the apparatus in adapter 13. Housing 22 is formed of plastic or similar material. Lip 23 is formed on the lower edge of housing 22 and contains O-ring 24. Housing 22 is attached to plug member 32 by rotatable fastening means formed by openings 40 and 41 and associated projections 42 and 43. Projections 42 and 43 extend from the sides of plug member 32 and fit into openings 40 and 41 formed in the housing 22. Plug member 32 may be retained or removed from housing 22 by turning and pulling or turning and pushing the plug member with respect to housing 22. This allows the portions of apparatus 1 carrying out the nebulizing of the liquid and positioned in cavity 33, to be removed at the end of therapy, for replacement, or for cleaning when a different drug is to be administered to the subject.

O-ring 24 seals the disc-like plate 25 to lip 23. Plate 25 is shown with enlarged thickness in Fig. 2a and may be formed of a conductive material such as brass. Plate 25 contains a central opening 26. Mesh plate 27 is mounted across central opening 26 of plate 25 by gluing with conducting glue, brazing, welding, or other suitable technique.

Mesh plate 27 is a relatively thin plate having a plurality of holes 28. Mesh plate 27 may be about 0.02 mm thick. The diameter of the holes at front surface 44 is preferably approximately 2-15 µm. Such holes may be formed in the plate by an electroforming process, which process produces holes of increasing diameter toward rear surface 45 of mesh plate 27. However, straight holes will work equally well, the primary criterion being that the exit diameter in front surface 44 of mesh plate 27 is such as to form droplets of the desired size.

Front surface 44 of mesh plate 27 is exposed to the pressure of the breathing gases in breathing circuit 2. These pressures will vary during inhalation and exhalation conditions in the breathing circuit. For example, with artificial ventilation, breathing circuit pressures may increase up to 100 mbar during inspiration and thereafter decrease during expiration. Disc-like plate 25 is provided with pressure balancing channel 37, as shown in Fig. 2a, that connects the volume between plug member 32 and plate 25 with breathing circuit 2 for equalizing the prevailing pressure at both sides of mesh plate 27. The pressure balancing provided by channel 37 prevents breathing gas from flowing through the holes in mesh plate 27, in opposition to the liquid being nebulized, which might otherwise degrade the operation of nebulizer apparatus 1 and avoids pressure stressing of mesh plate 27 and causing leaks to occur through the mesh plate.

In the nebulizer apparatus shown in Figs. 2a, 2b, and 2c, a vibrating element, such as a piezoelectric element 29, is mounted adjacent the surface of plate 25 which is the upper surface of the plate when the nebulizer is oriented as shown in Fig. 2a. Specifically, piezoelectric element 29 is spaced from plate 25 by a small gap 30 and secured to plate 25 about its periphery by a conductive glue, brazing, welding, or other suitable technique, shown as 31 in Figs. 2a, 2b, and 2c. Piezoelectric element 29 has a central opening corresponding to the central opening 26 of plate 25.

Plug member 32 is formed from a non-conductive material, such as plastic. Plug member 32 has a lower lip 34 containing an O-ring 35. Plug member 32 is placed on top of plate 25 so that the plate is between O-rings 24 and 35 when the plug member is placed in cavity 33 defined by housing 22.

An electric power terminal 38 extends through plug member 32. The lower end of terminal 38, which terminal may be in the form of a spring-loaded pin, contacts piezoelectric element 29. The upper end of terminal 38 is connected to cable 20 shown in Fig. 1. A second electrical power terminal 39 also extends through plug member 32. The lower end of terminal 39 contacts conductive plate 25. The upper end of terminal 39 is also connected to cable 20. Terminal 38, and terminal 39 which may be electrically grounded, apply a voltage to piezoelectric element 29. Grounded terminal 39 may also be used for capacitive measurement in conjunction with a plate 36 at the end of liquid transport line 17a which also functions as an electrical terminal and is hereinafter described in detail.

A central liquid transport line 17a extends through plug member 32 so that the plate 36 at the end of liquid transport line 17a is proximate to the upper surface of plate 25 and mesh plate 27. Plate 36 is formed of an electrically conductive material, but it is electrically isolated from its surroundings and from mesh plate 27 to allow its use in capacitance measurement of the presence of liquid in nebulizer apparatus 1.

In operation, valve 19 is opened in response to a signal from cable 20 and liquid flows through conduit 17 and transport line 17a due to the pressurizing gas in reservoir 18. The liquid flows out the end of transport line 17a as shown in Fig. 2a, into the volume between the plate 36 and the upper surface 45 of mesh plate 27. The cohesive forces in the liquid create a column of liquid between the plate 36 and mesh plate 27, as shown in Fig. 3.

To control the transport of liquid from reservoir 18 into the nebulizing apparatus 1, plate 36 and mesh plate 27 function as sensing electrodes that detect the presence and amount of the liquid supply between the plate 36 and the upper or rear surface 45 of mesh plate 27 by alteration of the capacitance between the two electrodes. Fig. 3 shows a more detailed picture of the capacitance electrodes and the behavior of the liquid between them. In Fig. 3 $R_f$ is a radius of liquid column 50 between plate 36 and mesh plate 27, $R_p$ is a radius of plate 36 and L is a distance between the two plates.

The capacitance existing between the plate 36 and mesh plate 27 is dependent on the different variables present, as follows:

$$C = \frac{\varepsilon_0 \cdot \pi \cdot [\varepsilon_{air} \cdot R_p^2 - \varepsilon_{air} \cdot R_f^2 + \varepsilon_{liquid} \cdot R_f^2]}{L}$$

where

$\varepsilon_0$ = dielectric coefficient of a vacuum
$\varepsilon_{air}$ = dielectric coefficient of the air between the two plates 27, 36
$\varepsilon_{liquid}$ = dielectric coefficient of the liquid between the two plates
$R_p$ = radius of the plate 36
$R_f$= radius of the liquid column 50 between the two plates 27, 36
L = distance between the two plates 27, 36

The dielectric coefficient of the liquid to be nebulized is much higher than the dielectric coefficient of the air and enables the amount of liquid or the radius of the liquid column to be measured accurately between the two plates.

In order for the capacitive measurement to function properly, the distance between plate 36 and mesh plate 27 has to be adjusted to a suitable distance. If the distance between the two plates is too great, the capacitance values between the plates becomes too low to obtain an accurate measurement result. If the distance between the plates is too little, the operation of the nebulizer apparatus may be adversely affected, as noted below.

Reliable results can be obtained when capacitance values vary over a range of a few pF and this will be obtained with radius $R_p$ of 2.5 mm for plate 36 and a distance L of 0.5 mm between the plate 36 and mesh plate 27.

The area of the plate 36 also has an effect on sensitivity of the capacitive measurement. Shape of the area of plate 36 can have different forms. While a rounded or a square shape for plate 36 is easier to analyze, other forms such as triangular, rectangular, star, or other multi-sided configurations may be used A round plate is discussed below for explanatory purposes. If the radius of the plate 36 is increased, the overlap area of the electrodes becomes larger and higher values of capacitance will be achieved. The radius of the plate 36 can also be made smaller, but the distance between the plate 36 and mesh plate 27 must be decreased at the same time to maintain a certain level of capacitance.

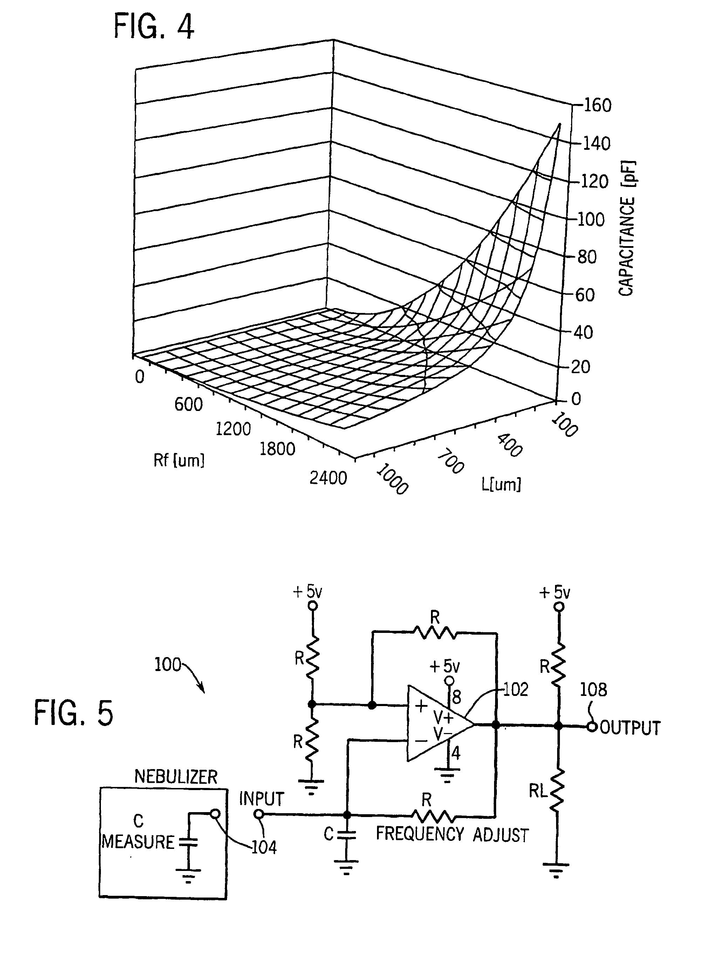

Fig. 4 shows the dependence of capacitance in pico farads on the z-axis with respect to the radius $R_f$ of liquid column in micrometers on the x-axis and the distance L between the plates 27 and 36 in micrometers on the y-axis. It can be seen that both increasing the radius of liquid column $R_f$, in other words increasing the overlapping area of plates 27 and 36 where the liquid column may exist and decreasing the distance L between the plates 27 and 36 increase the measured values of capacitance fairly exponentially.

With a continued supply of liquid, the liquid column 50 between the plate 36 and the rear surface 45 of mesh plate 27 will grow in the radial direction and significantly increase the capacitance measured between the two plates. The capacitance between the two sensing electrodes is inputted to sensor circuitry inside the nebulizer 1 or inside the control unit 4 via cable 20 and is used by the control unit 4 to close the valve 19 in the liquid supply conduit 17.

When the capacitance decreases due to the liquid receding away between plate 36 and mesh plate 27 during operation of nebulizer apparatus 1, the control valve 19 is opened to allow flow of liquid from the end of transport line 17a. The delivery of nebulized liquid can be carried out responsive to the activation of the vibration of mesh plate 27 by intermittently supplying liquid when the amount of liquid between the mesh plate 27 and the plate 36 is reduced.

Fig. 5 shows an example of a circuit 100 to measure capacitance between plates 27 and 36 serving as the sensing electrodes. In this circuit, element 102 may comprise a capacitively controlled RC-oscillator, the output of which is a continuous train of pulses. Change in capacitance between elements 27 and 36, as reflected in the signal in conductor 104, changes the frequency or pulse width of the output. The continuous pulse train may be low pass filtered by a filter (not shown) to provide a DC-signal at output 108 having a linear response with respect to measured capacitance. When the capacitive measurement is carried out during the vibrating of mesh plate 27, these vibrations cause the capacitance between elements 27 and 36 to alter in phase with the vibration. The result is a modulation of the capacitance related output signal by a varying signal. Additional filtering may be employed to filter out the varying signal produced by the vibrations of mesh plate 27.

In operation, alternating voltage is supplied from a power source inside the control unit 4 though cable 20 and terminals 38 and 39 to piezoelectric element 29, which vibrates the element. The alternating voltage causes the element 29 to contract from the normal condition, shown in Fig. 2b, to a radially decreased condition shown in Fig. 2c and then return to the normal condition. Due to the joinder of piezoelectric element 29 to plate 25 about the periphery of the element, the radial size reduction of piezoelectric element 29 causes plate 25 to bow, as shown in Fig. 2c, and then return to the flat condition, shown in Fig. 2b, when piezoelectric element 29 returns to the normal state. The action of plate 25, as shown in Figs. 2b and 2c moves liquid fed to the upper surface 45 of mesh plate 27 through holes 28 onto the front surface 44 of mesh plate 27 by inertia and the motion of the liquid in phase difference compared to the vibration of mesh plate 27. At the front surface 44 of the vibrating mesh plate 27, the atomized liquid will grow into drops at each hole 28 due to the liquid surface tension. The drops will increase in size until the expelling forces arising from the movement of mesh plate 27 and the mass of each drop exceeds the holding force determined by the size of the holes 28 in mesh plate 27 and the surface tension of the liquid at which point the drop is expelled. The drops expelled from mesh plate 27 pass through the housing 22 into the patient limb 10, and to the subject during inhalation. As noted above, the action of plate 25, as shown in Figs. 2b and 2c also has an effect on capacitive measurement due to the variation of distance L between the plate 36 and mesh plate 27, but this interference, having a frequency of mesh plate vibration, can be filtered from the output signal of the circuitry shown in Fig. 5.

The sensitivity of capacitive measurement becomes better as the distance L between the two plates becomes smaller, as can be seen from Fig. 4. However the distance L between plates 27 and 36 should not be too small. If the distance between the plates becomes too small, the amount of liquid is greatly reduced and the compressing force directed to the liquid caused by the bowing action of vibrating plate 25 with respect to plate 36 is increased. This compressing force directed to the liquid will squeeze the liquid under the pressure through the holes 28 in mesh plate 27. This squeezing action differs from the desired action where the discharging of liquid through the holes of the mesh plate is caused by the inertia and the motion of the liquid in phase difference compared to vibrating mesh plate. At some frequencies this squeezing action may work equally well as the desired action, but at frequencies where the desired action occurs it may damp the vibrations of plate 25 and interfere with the functioning of mesh plate 27 preventing the droplets from being formed.

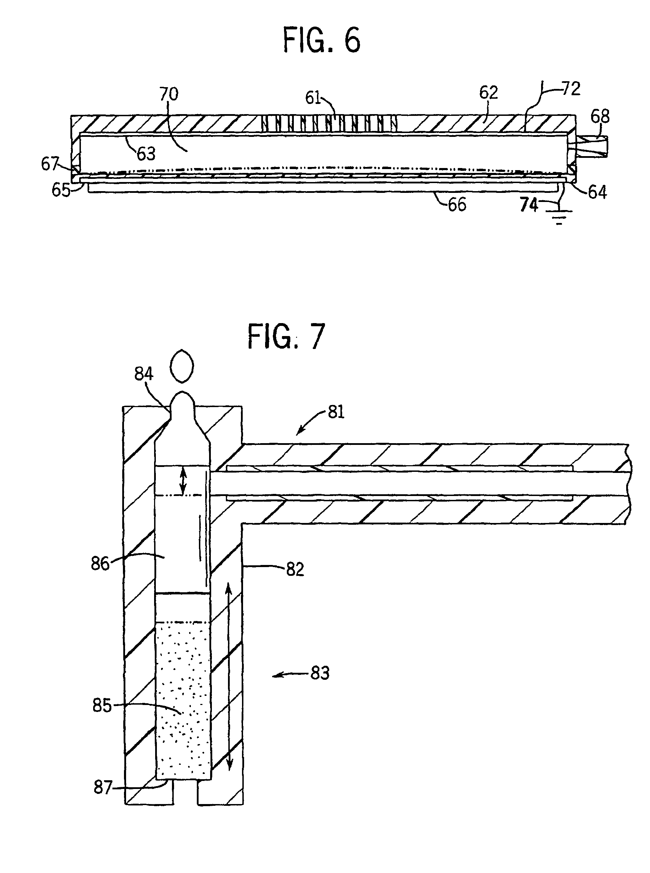

Fig. 6 shows an example of another type of nebulizer apparatus construction having capacitive measurement to control the supply of liquid for the atomizing means. In this construction cover 62, which may be of plastic or silicone, and bottom part 64, which is a bimorph, are closed tightly together with an elastic joint 67 to form a chamber 70 for the liquid to be nebulized. A nozzle assembly 61, such as a mesh plate, is glued into a hole in the middle of the surface of cover 62 or, alternatively, a nozzle or nozzles can be integrated directly into cover 62, as shown in Fig. 6. The lower surface of cover 62, when the nebulizer apparatus is oriented as shown in Fig. 6, has an electrically conductive coating 63, which functions as a sensing electrode for capacitive measurement, via conductor 72. The bottom part 64, which is a bimorph, is composed of piezoelectric crystal 66 attached to metal plate 65. Metal plate 65, which is the upper surface of bottom part 64, functions as a counterpart for the sensing electrode 63. To ensure that the capacitive measurement functions properly, sensing electrode 63 and the upper surface of bottom part 64 must be electrically isolated from each other. This can be achieved by providing an electrically isolating coating on both or at least on one of these electrically conductive surfaces. Vibrating element 66, such as a piezoelectric crystal, which is operated in the radial mode, shown in Fig. 2b and 2c, is mounted on the lower surface of metal plate 65 by electrically conductive glue or other suitable technique. This enables the metal plate 65 to also function as an electrical ground for the vibrating element 66 via conductor 74. Tube 68 extends through the side wall of cover 62 so that the chamber 70 can be filled with liquid. Tube 68 is tapered toward the chamber and functions as a diffuser, preventing reverse flow of the liquid.

In operation, an alternating voltage supplied to vibrating element 66 forces the bottom part 64 to vibrate between two states. In one state, vibrating element 66 causes bottom part 64 to bend, as shown with a dotted line in the Fig. 6. The other state is the normal flat state. The inward bending of bottom part 64 decreases the volume of chamber 70 and increases the pressure on the liquid inside it. The increase in pressure, when applied to the liquid inside the chamber forces the liquid through the nozzle or nozzles 61 placed in the cover 62. As the aerosol is generated, air may flow through the nozzle or nozzles into the chamber 70 due the under pressure formed when the bottom part 64 returns to the normal state. The air inside the chamber will hamper the generation of aerosol because the pressure generated during the bent state of bottom part 64 has an effect mostly on the air due to its higher compressibility as compared to liquid. The amount of air flowing into chamber 70 and the emptying of chamber 70 of liquid can be measured with the capacitive measurement between coatings 63 and 65 and more liquid can be fed to chamber 70 through tube 68 to keep the chamber filled.

Fig. 7 shows a further embodiment of the improved device for measuring the amount of liquid to be discharged by an apparatus. In contrast to the embodiments previously described, the measuring device of Fig. 7 is located in the transport line that supplies the liquid to be discharged from a reservoir to the apparatus rather than in a cavity within the apparatus. Specifically, and as shown in Fig. 7, liquid to be discharged is supplied in transport line 81 to housing 82 of discharge apparatus 83. Discharge apparatus 83 includes one or more nozzles 84, in the housing. Housing 82 contains piezoelectric element 85 attached to piston 86 and to the bottom wall 87 of housing 82. When piezoelectric element 85 is energized by high frequency alternating current, piston 86 alternately moves between the position shown by the dashed line in Fig. 7 and the position shown by the solid line. When piston 86 is in the position shown by the dashed line, liquid from transport line 81 enters housing 82. When piston 86 moves to the position shown in the solid line, liquid in housing 82 is discharged through nozzle 84.

To control the supply of liquid to housing 82 of discharge apparatus 83, two opposite walls 87 and 88 of transport line 81 are provided with electrically conductive members 89 and 90, respectively. Members 89 and 90 function as sensing electrodes for capacitive measurement of the presence and amount of liquid in transport line 81 in the same manner as described above. The measurement carried out by members 89 and 90 can be used to control the provision of liquid to transport line 81 to ensure that the transport line is full of liquid.

The above described technique of capacitive measurement is particularly suitable for micro mechanical apparatuses using elements formed of silicon.

It is recognized that other equivalents, alternatives, and modifications aside from those expressly stated, are possible and within the scope of the appended claims.

## Claims

1. A nebulizing apparatus for nebulizing a liquid comprising:

   a housing (22);
   a chamber in said housing for receiving liquid to be nebulized;
   a first member (27) mounted to said housing and having holes through which the liquid passes when nebulized said chamber being in fluid communication with said first member;
   a second member (36) mounted to said housing and spaced from said first member so that a volume (50) is defined in the chamber by the area of mutual overlap of said first and second members and the amount of spacing between said first and second members in the area of overlap, said first and second members being formed to establish a mutual capacitance that reflects the amount of liquid present in the volume;
   means (17a) for providing liquid into the volume between said first and second members;
   a vibrator (29) for creating vibratory pressures in the liquid for causing the liquid to pass through the holes in said first member; and
   means (100) for measuring the capacitance existing between said first and second members and hence the amount of liquid in the nebulizer, said means being coupled to said liquid providing means for controlling the provision of liquid into the volume in accordance with the capacitance measured by said measuring means.

2. The apparatus according to claim 1 wherein said first member (27) is a plate having a plurality of holes.

3. The apparatus according to claim 2 wherein said plate (27) is formed of conductive material.

4. The apparatus according to any preceding claim wherein said liquid providing means (17a) is coupled to said second member (36) for providing liquid into the volume (50).

5. The apparatus according to any preceding claim further including a vibrator operatively associated with one of said first and second members.

6. The apparatus according to any preceding claim wherein said measuring means (100) is coupled to said liquid providing means (17a) for causing said liquid providing means to provide liquid into the volume (50) responsive to the measurement of the capacitance existing between said first and second members (27, 36).

7. The apparatus according to any preceding claim wherein said liquid providing means (17a) is further defined as intermittently providing liquid into the volume between the first and second members.

8. An apparatus for discharging liquid which includes a liquid transport line (81), the apparatus comprising a device for measuring the amount of liquid to be discharged that is present in the liquid transport line for the apparatus, said device comprising:

a first member (89) in the transport line and a second member (90) in the transport line spaced from said first member so that a volume is defined in the transport line by the area of mutual overlap of said first and second members and the amount of spacing between said first and second members in the area of overlap, said first and second members (89, 90) being formed to establish a mutual capacitance that reflects the amount of liquid present in the volume; and
means for measuring the capacitance existing between said first and second members (89, 90) and hence the amount of liquid in the transport line (81).

9. The apparatus according to any preceding claim wherein at least one of said first and second member (27, 36; 89, 90) is formed of conductive material.

10. The apparatus according to any preceding claim wherein said first and second members (27, 36; 89, 90) have electrically conductive properties, said first and second members being electrically isolated from each other.

11. The apparatus according to any preceding claim wherein at least one of said first and second member (27, 36; 89, 90) has an electrically conductive coating.

12. The apparatus according to any preceding claim wherein at least one of said first and second members (27, 36) is round.

13. The apparatus according to any preceding claim wherein said means (100) for measuring the capacitance existing between said first and second members comprises a capacitively controlled RC-oscillator receiving an input from at least one of said first and second members (27, 36; 89, 90) and providing a pulse train output, and filter means for filtering said pulse train output to provide a signal indicative of the capacitance existing between said first and second members.

14. The apparatus according to claim 13 wherein said measuring means is coupled to said liquid providing means (17a) for controlling the provision of liquid into the volume.

15. The apparatus according to claim 8 further defined as an apparatus including a device for measuring the amount of liquid that is present in the liquid transport line for a nebulizer.

**Patentansprüche**

1. Eine Zerstäubvorrichtung zum Zerstäuben einer Flüssigkeit, die folgende Merkmale aufweist:

   ein Gehäuse (22);

   eine Kammer in dem Gehäuse zum Aufnehmen einer zu zerstäubenden Flüssigkeit;

   ein erstes Bauglied (27), das an dem Gehäuse befestigt ist und Löcher aufweist, durch die die Flüssigkeit hindurchtritt, wenn dieselbe zerstäubt ist, wobei die Kammer in Fluidkommunikation mit dem ersten Bauglied steht;

   ein zweites Bauglied (36), das an dem Gehäuse befestigt ist und so von dem ersten Bauglied beabstandet ist, dass ein Volumen (50) in der Kammer definiert ist durch die Fläche einer gegenseitigen Überlappung des ersten und des zweiten Bauglieds und den Grad der Beabstandung zwischen dem ersten und dem zweiten Bauglied in der Überlappungsfläche, wobei das erste und das zweite Bauglied gebildet sind, um eine gegenseitige Kapazität einzurichten, die die Flüssigkeitsmenge, die in dem Volumen vorhanden ist, widerspiegelt;

   eine Einrichtung (17a) zum Bereitstellen von Flüssigkeit in das Volumen zwischen dem ersten und dem zweiten Bauglied;

   einen Vibrator (29) zum Erzeugen von Vibrationssdrücken in der Flüssigkeit, um zu bewirken, dass die Flüssigkeit durch die Löcher in dem ersten Bauglied hindurchtritt; und

   eine Einrichtung (100) zum Messen der Kapazität, die zwischen dem ersten und dem zweiten Bauglied besteht, und damit der Flüssigkeitsmenge in dem Zerstäuber, wobei die Einrichtung mit der Flüssigkeitbereitstelleinrichtung zum Steuern der Bereitstellung von Flüssigkeit in das Volumen gemäß der Kapazität, die durch die Messeinrichtung gemessen wird, gekoppelt ist.

2. Die Vorrichtung gemäß Anspruch 1, bei der das erste Bauglied (27) eine Platte mit einer Mehrzahl von Löchern ist.

3. Die Vorrichtung gemäß Anspruch 2, bei der die Platte (27) aus einem leitfähigen Material gebildet ist.

4. Die Vorrichtung gemäß einem der vorhergehenden Ansprüche, bei der die Flüssigkeitbereitstelleinrichtung (17a) mit dem zweiten Bauglied (36) zum Bereitstellen von Flüssigkeit in das Volumen (50) gekoppelt ist.

5. Die Vorrichtung gemäß einem der vorhergehenden Ansprüche, die ferner einen Vibrator umfasst, der wirksam entweder dem ersten oder dem zweiten Bauglied zugeordnet ist.

6. Die Vorrichtung gemäß einem der vorhergehenden Ansprüche, bei der die Messeinrichtung (100) mit der Flüssigkeitbereitstelleinrichtung (17a) gekoppelt ist, um zu bewirken, dass die Flüssigkeitbereitstelleinrichtung Flüssigkeit ansprechend auf die Messung der Kapazität, die zwischen dem ersten und dem zweiten Bauglied (27, 36) besteht, in das Volumen (50) bereitstellt.

7. Die Vorrichtung gemäß einem der vorhergehenden Ansprüche, bei der die Flüssigkeitbereitstelleinrichtung (17a) ferner so definiert ist, dass dieselbe intermittierend Flüssigkeit in das Volumen zwischen dem ersten und dem zweiten Bauglied bereitstellt.

8. Eine Vorrichtung zum Abgeben von Flüssigkeit, die eine Flüssigkeitstransportleitung (81) umfasst, wobei die Vorrichtung ein Gerät zum Messen der Menge an auszustoßender Flüssigkeit aufweist, die in der Flüssigkeitstransportleitung für die Vorrichtung vorhanden ist, wobei das Gerät folgende Merkmale aufweist:

ein erstes Bauglied (89) in der Transportleitung und ein zweites Bauglied (90) in der Transportleitung, das von dem ersten Bauglied so beabstandet ist, dass ein Volumen in der Transportleitung durch die Fläche einer gegenseitigen Überlappung des ersten und des zweiten Bauglieds und den Grad der Beabstandung zwischen dem ersten und dem zweiten Bauglied in der Überlappungsfläche definiert ist, wobei das erste und das zweite Bauglied (89, 90) gebildet sind, um eine gegenseitige Kapazität einzurichten, die die Flüssigkeitsmenge, die in dem Volumen vorhanden ist, widerspiegelt; und

eine Einrichtung zum Messen der Kapazität, die zwischen dem ersten und dem zweiten Bauglied (89, 90) besteht, und damit der Flüssigkeitsmenge in der Transportleitung (81).

9. Die Vorrichtung gemäß einem der vorhergehenden Ansprüche, bei der zumindest entweder das erste oder das zweite Bauglied (27, 36; 89, 90) aus leitfähigem Material gebildet ist.

10. Die Vorrichtung gemäß einem der vorhergehenden Ansprüche, bei der das erste und das zweite Bauglied (27, 36; 89, 90) elektrisch leitfähige Eigenschaften aufweisen, wobei das erste und das zweite Bauglied elektrisch voneinander getrennt sind.

11. Die Vorrichtung gemäß einem der vorhergehenden Ansprüche, bei der zumindest entweder das erste oder das zweite Bauglied (27, 36; 89, 90) eine elektrisch leitfähige Beschichtung aufweist.

12. Die Vorrichtung gemäß einem der vorhergehenden Ansprüche, bei der zumindest entweder das erste oder das zweite Bauglied (27, 36) rund ist.

13. Die Vorrichtung gemäß einem der vorhergehenden Ansprüche, bei der die Einrichtung (100) zum Messen der Kapazität, die zwischen dem ersten und dem zweiten Bauglied besteht, einen kapazitiv gesteuerten RC-Oszillator, der ein Eingangssignal von zumindest entweder dem ersten oder dem zweiten Bauglied (27, 36; 89, 90) empfängt und ein Pulsfolgeausgangssignal bereitstellt, sowie eine Filtereinrichtung zum Filtern des Pulsfolgeausgangssignals aufweist, um ein Signal bereitzustellen, das die Kapazität, die zwischen dem ersten und dem zweiten Bauglied besteht, anzeigt.

14. Die Vorrichtung gemäß Anspruch 13, bei der die Messeinrichtung mit der Flüssigkeitbereitstelleinrichtung (17a) zum Steuern der Bereitstellung von Flüssigkeit in das Volumen gekoppelt ist.

15. Die Vorrichtung gemäß Anspruch 8, die ferner definiert ist als eine Vorrichtung, die ein Gerät zum Messen der Flüssigkeitsmenge, die in der Flüssigkeitstransportleitung für einen Zerstäuber vorhanden ist, umfasst.

## Revendications

1. Appareil nébuliseur pour nébuliser un liquide comprenant :

un boîtier (22) ;
une chambre dans ledit boîtier pour recevoir le liquide à nébuliser ;
un premier élément (27) monté sur ledit boîtier et ayant des trous à travers lesquels le liquide passe lorsqu'il est nébulisé, ladite chambre étant en communication fluide avec ledit premier élément ;
un deuxième élément (36) monté sur ledit boîtier et écarté dudit premier élément de façon à ce qu'un volume (50) soit défini dans la chambre par la zone de chevauchement mutuel desdits premier et deuxième éléments et la grandeur de l'écartement entre lesdits premier et deuxième éléments dans la zone de chevauchement, lesdits premier et deuxième éléments étant formés de façon à établir une capacité mutuelle qui reflète la quantité de liquide présente dans le volume ;

un moyen (17a) pour fournir du liquide dans le volume entre lesdits premier et deuxième éléments ;

un vibreur (29) pour créer des pressions vibratoires dans le liquide afin de faire passer le liquide à travers les trous dans ledit premier élément ; et

un moyen (100) pour mesurer la capacité existant entre lesdits premier et deuxième éléments et, par conséquent, la quantité de liquide dans le nébuliseur, ledit moyen étant couplé audit moyen de fourniture de liquide afin de contrôler la fourniture de liquide dans le volume selon la capacité mesurée par ledit moyen de mesure.

2. L'appareil selon la revendication 1, dans lequel ledit premier élément (27) est une plaque comportant plusieurs trous.

3. L'appareil selon la revendication 2, dans lequel ladite plaque (27) est formée d'un matière conductrice.

4. L'appareil selon l'une quelconque des revendications précédentes dans lequel ledit moyen de fourniture de liquide (17a) est couplé audit deuxième élément (36) pour fournir du liquide dans le volume (50).

5. L'appareil selon l'une quelconque des revendications précédentes qui comprend également un vibreur associé opérationnellement à un desdits premier et deuxième éléments.

6. L'appareil selon l'une quelconque des revendications précédentes dans lequel ledit moyen de mesure (100) est couplé audit moyen de fourniture de liquide (17a) pour faire que ledit moyen de fourniture de liquide fournisse du liquide dans le volume (50) en réponse à la mesure de la capacité existant entre lesdits premier et deuxième éléments (27, 36).

7. L'appareil selon l'une quelconque des revendications précédentes dans lequel ledit moyen de fourniture de liquide (17a) est défini en outre comme fournissant du liquide de façon intermittente dans le volume entre lesdits premier et deuxième éléments.

8. Appareil pour décharger du liquide qui comprend une conduite de transport de liquide (81), cet appareil comprenant un dispositif pour mesurer la quantité de liquide à décharger qui est présente dans la conduite de transport de liquide pour l'appareil, ledit dispositif comprenant :

un premier élément (89) dans la conduite de transport et un deuxième élément (90) dans la conduite de transport écarté dudit premier élément de façon à ce qu'un volume soit défini dans la conduite de transport par la zone de chevauchement mutuel desdits premier et deuxième éléments et la grandeur de l'écartement entre lesdits premier et deuxième éléments dans la zone de chevauchement, lesdits premier et deuxième éléments (89, 90) étant formés de façon à établir une capacité mutuelle qui reflète la quantité de liquide présente dans le volume ; et

un moyen pour mesurer la capacité existant entre lesdits premier et deuxième éléments (89, 90) et par conséquent la quantité de liquide dans la conduite de transport (81).

9. L'appareil selon l'une quelconque des revendications précédentes dans lequel au moins un desdits premiers et deuxièmes éléments (27, 36 ; 89, 90) est formé d'une matière conductrice.

10. L'appareil selon l'une quelconque des revendications précédentes dans lequel lesdits premiers et deuxièmes éléments (27, 36 ; 89, 90) ont des propriétés conductrices d'électricité, lesdits premiers et deuxièmes éléments étant isolés électriquement les uns des autres.

11. L'appareil selon l'une quelconque des revendications précédentes dans lequel au moins un desdits premiers et deuxièmes éléments (27, 36 ; 89, 90) a un revêtement conducteur d'électricité.

12. L'appareil selon l'une quelconque des revendications précédentes dans lequel au moins un desdits premiers et deuxièmes éléments (27, 36) est rond.

13. L'appareil selon l'une quelconque des revendications précédentes dans lequel ledit moyen (100) pour mesurer la capacité existant entre lesdits premiers et deuxièmes éléments comprend un oscillateur RC commandé par capacité qui reçoit une entrée venant d'au moins un desdits premiers et deuxièmes éléments (27, 36 ; 89, 90) et qui fournit un train d'impulsions de sortie, et un moyen filtre pour filtrer ledit train d'impulsions de sortie afin de fournir un signal indicatif de la capacité existant entre lesdits premiers et deuxièmes éléments.

14. L'appareil selon la revendication 13 dans lequel ledit moyen de mesure est couplé audit moyen de fourniture de liquide (17a) pour commander la fourniture de liquide dans le volume.

**15.** L'appareil selon la revendication 8 défini en outre comme un appareil comprenant un dispositif pour mesurer la quantité de liquide qui est présente dans la conduite de transport de liquide pour un nébuliseur.

FIG. 1

FIG. 2a

# FIG. 2b

NORMAL CONDITION

31  29

25  27

# FIG. 2c

DIRECTION OF BENDING

DIRECTION OF RADIAL DECREASE

29  31

25  27

# FIG. 3

LIQUID

CAPACITANCE MEASUREMENT

17a

$R_p$

36

20

$R_f$

45

$R'_f$

$L$

27

50

ATOMIZED LIQUID

FIG. 4

FIG. 5

# FIG. 6

# FIG. 7